# EUROPEAN PATENT APPLICATION

(11) **EP 4 552 623 A1**
(43) Date of publication of application: **14.05.2025**
(21) Application number: 23835776.8
(22) Date of filing: 03.07.2023
(51) Int. Cl.: A61F 9/007, A61F 9/00

(54) **SHUNT FOR FIBROUS ENCAPSULATION IN GLAUCOMA PATIENTS**

(30) Priority: 05.07.2022 KR 20220082539
(71) Applicant: Seoul National University R & DB Foundation, Seoul 08826 (KR)
(72) Inventor: KANG, Seon Mi, Seongnam-si, Gyeonggi-do 13316 (KR); SEO, Kang Moon, Seoul 03302 (KR); LEE, Song Hui, Seoul 06683 (KR)
(74) Representative: Schön, Christoph
(86) International application number: PCT/KR2023/009331
(87) International publication number: WO 2024/010310

(57) **Abstract**

This disclosure relates to a shunt for fibrous encapsulation of glaucoma patients which lowers intraocular pressure by penetrating a fibrous encapsulation of a bleb formed between a sclera and a conjunctiva and draining aqueous humor within the bleb to subconjunctival.

## Description

### [Technical Field]

The present disclosure relates to a shunt for fibrous encapsulation of glaucoma patients. More particularly, the present disclosure relates to a shunt which is mounted in a fibrous encapsulation formed at a surgical site after a glaucoma surgery, such as trabeculectomy and glaucoma drainage device implantation, and drains aqueous humor within the fibrous encapsulation.

### [Background Art]

Glaucoma is an eye disease in which the circulation of aqueous humor is abnormal, causing an increase in an amount of the aqueous humor in the eye and an increase in intraocular pressure. When the intraocular pressure increases due to glaucoma, the optic nerve is compressed and the blood flow to the eye decreases, causing degeneration of the optic nerve and retina. This may result in vision loss or visual field defects and may be accompanied by severe pain.

Because an increase in progressive intraocular pressure due to aqueous humor drainage disorder causes irreversible vision loss, attempts are needed to maintain the intraocular pressure at normal levels. To this end, glaucoma can be managed through pharmacological treatment and surgical treatment.

The pharmacological treatment is performed using drugs which suppress the production of aqueous humor or increase the drainage of aqueous humor. When treating glaucoma, the pharmacological treatment is generally performed first, and the purpose of pharmacological treatment is to slow the progression of vision loss due to glaucoma. However, the pharmacological treatment has the disadvantage of being difficult to sufficiently alleviate the increase in intraocular pressure caused by glaucoma as glaucoma progresses, and a high incidence of side effects to the drug (conjunctivitis, uveitis, hypersensitivity of the skin around the eye, or the like).

Among various glaucoma surgeries, the surgical treatment as a method of increasing aqueous humor drainage involves a surgery to divert aqueous humor, which is not drained from an anterior chamber of the eye, to subconjunctival or subtenon to thereby allow the aqueous humor to be absorbed through a route other than the eyeball. The surgical treatment includes trabeculectomy, glaucoma drainage device implantation, or the like.

The trabeculectomy is a surgery to drain aqueous humor by creating an aqueous humor bypass without implantation of a device.

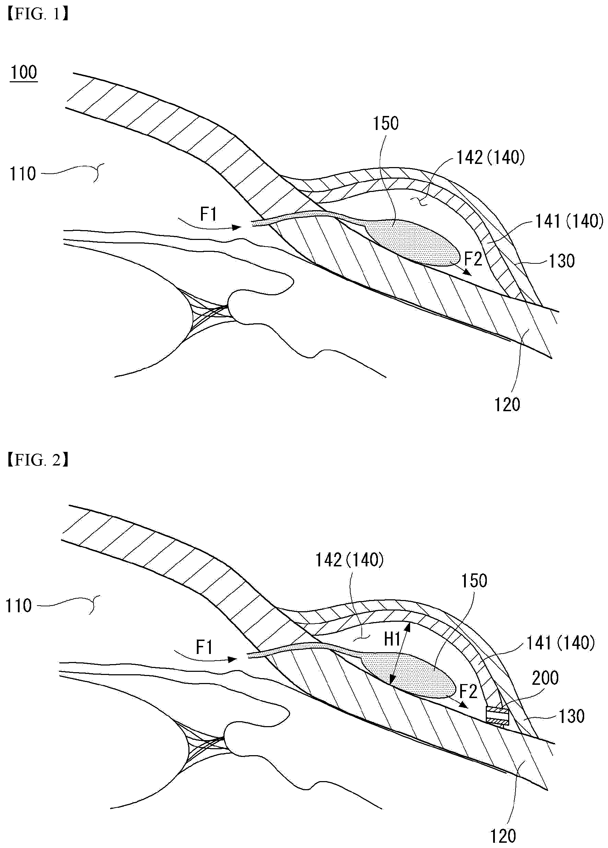

As illustrated in FIG. 1, the glaucoma drainage device implantation is a surgery to drain an aqueous humor of an anterior chamber 110 by implanting a glaucoma drainage device 150 including an aqueous humor bypass.

A side of the glaucoma drainage device 150 may be located in the anterior chamber 110. The side of the glaucoma drainage device 150 may be in the form of a tube. Aqueous humor in the anterior chamber 110 may flow into the side of the glaucoma drainage device 150. A direction in which the aqueous humor of the anterior chamber 110 flows into the glaucoma drainage device 150 may be referred to as a first direction F1.

Another side of the glaucoma drainage device 150 may be located outside a sclera 120. The aqueous humor within the glaucoma drainage device 150 may be drained to the other side of the glaucoma drainage device 150. A direction in which the aqueous humor within the glaucoma drainage device 150 is drained to a space between the sclera 120 and a conjunctiva 130 may be referred to as a second direction F2.

After the glaucoma drainage device 150 is implanted, a bleb may be formed around the glaucoma drainage device 150 after several weeks. The bleb around the glaucoma drainage device 150 may be referred to as a first bleb 140. The aqueous humor widely spread by the first bleb 140 may be absorbed into subconjunctival or subtenon and reabsorbed into the blood flow.

Fibrosis of the first bleb 140 may progress over time. An encapsulation of the fibrosed first bleb 140 may be referred to as a fibrous encapsulation. The causes of fibrosis of the first bleb 140 include a reaction to surgical stimulation, a preservative of antiglaucoma eye drops used before and after surgery, an inflammatory factor inherent in aqueous humor of a glaucoma patient, a reaction to stimulation according to the flow of aqueous humor, or the like.

Because the fibrosed first bleb 140 impedes the flow of the aqueous humor, the aqueous humor within the first bleb 140 cannot escape, which may cause the first bleb 140 to swell. This is a main cause of increased intraocular pressure. To deal with this, methods, such as using drugs which delay the fibrosis of the first bleb 140 during or after surgery, surgically removing an already fibrosed part, or implanting the glaucoma drainage device 150 into another part of the anterior chamber 110, are implemented.

The method of delaying the fibrosis of the first bleb 140 using the drugs has a problem of causing side effects (conjunctival ulcer, cataract, or the like) to the drugs themselves.

Because a surgery to remove a fibrous tissue of the fibrosed first bleb 140 is highly invasive, there are problems, such as slow patient recovery and a high possibility of other complications due to postoperative infection or weakened conjunctiva. In addition, when the fibrous tissue of the first bleb 140 is removed, there is a possibility that faster fibrosis may occur due to the biological characteristics, so symptoms after the surgery may worsen.

The method of implanting the glaucoma drainage device 150 into another part of the anterior chamber 110 is a method of performing an additional surgery on an eyeball 100 which has already experienced an inflammatory reaction, and thus there is a problem that there is a high risk of inflammation or infection. In addition, because the implantation of the glaucoma drainage device 150 is performed under general anesthesia for a long time, a physical burden on the patient may increase, and the time and financial burden of the surgery may increase.

Accordingly, there is a need for a device which can safely and effectively drain the aqueous humor within the first bleb 140 using a minimally invasive method.

An object of Korean patent application publication No. 10-2007-0035529 entitled 'Ophthalmic implant, method of manufacturing the same, and method of using the same' (hereinafter, 'patent document 1'), Korean patent application publication No. 10-2020-0118291 entitled 'Shunt stent for eyeball drainage' (hereinafter, 'patent document 2'), and Korean patent application publication No. 10-2022-0000690 entitled 'Glaucoma drainage device with adjustable tube diameter' (hereinafter, 'patent document 3') is to divert the aqueous humor of the anterior chamber 110 to subconjunctival and drain the aqueous humor.

Because the devices of the patent documents 1 to 3 are mounted on the eyeball 100, they are formed in a shape suitable for mounting on the spherical eyeball 100. The devices of the patent documents 1 to 3 are mounted between the inside of the spherical eyeball 100 and the subconjunctival.

Unlike the shape of the eyeball 100, the first bleb 140 is formed in a convex shape protruding from the sclera 120, and has a feature in that a height protruding from the sclera 120 decreases and the curvature decreases as the aqueous humor within the first bleb 140 is drained. The shape of the first bleb 140 is formed in an identical or similar shape regardless of a type of the glaucoma drainage device 150 mounted in the eyeball 100.

As described above, because the eyeball 100 and the first bleb 140 are different from each other in the shape and the feature, a device suitable for mounting in the first bleb 140 is required.

### [Disclosure]

### [Technical Problem]

An object of the present disclosure is to address the above-described and other problems.

Another object of the present disclosure is to minimally invasively drain aqueous humor within a bleb.

Another object of the present disclosure is to provide a shunt for preventing conjunctival thinning.

Another object of the present disclosure is to provide a shunt for smoothly draining aqueous humor.

Another object of the present disclosure is to provide a shunt which can be mounted in plural.

### [Technical Solution]

There may be provided a shunt comprising a main body penetrating a fibrous encapsulation of a bleb formed between a sclera and a conjunctiva and a flow path penetrating the main body and draining an aqueous humor within the bleb to an outside of the bleb.

The shunt may further comprise a first support member connected to a front of the main body and positioned outside the fibrous encapsulation, and a second support member connected to a rear of the main body and positioned inside the fibrous encapsulation. The main body may include a lower surface facing the sclera, and the flow path may penetrate the main body from the front to the rear.

### [Advantageous Effects]

Effects of a shunt for fibrous encapsulation of glaucoma patients according to an embodiment of the present disclosure are described as follows.

According to at least one of embodiments of the present disclosure, there may be provided a shunt which can minimally invasively drain aqueous humor within a bleb.

According to at least one of embodiments of the present disclosure, there may be provided a shunt which prevents conjunctival thinning.

According to at least one of embodiments of the present disclosure, there may be provided a shunt which smoothly drains aqueous humor.

According to at least one of embodiments of the present disclosure, there may be provided a shunt which that can be mounted in a fibrous encapsulation in plural.

Additional scope of applicability of the present disclosure will become apparent from the detailed description given blow. However, it should be understood that the detailed description and specific examples such as embodiments of the present disclosure are given merely by way of example, since various changes and modifications within the spirit and scope of the present disclosure will become apparent to those skilled in the art from the detailed description.

### [Description of Drawings]

FIG. 1 illustrates an eyeball 100 in which a first bleb 140 is formed around a glaucoma drainage device 150.
FIG. 2 illustrates that a shunt 200 according to an embodiment of the present disclosure is mounted in the first bleb 140 of FIG. 1.
FIG. 3 is a perspective view illustrating the shunt 200 of FIG. 2.
FIG. 4 is a plan view of the shunt 200 of FIG. 3.
FIG. 5 is a plan view illustrating a shunt 1200 according to another embodiment of the present disclosure.
FIG. 6 is a front view illustrating a shunt 2200 according to another embodiment of the present disclosure.
FIG. 7 illustrates that aqueous humor within a first bleb 140 of FIG. 2 is drained through a shunt 200.
FIG. 8 illustrates that a second bleb 170 is formed due to aqueous humor drained through the shunt 200 of FIG. 7.
FIG. 9 illustrates that an additional shunt 200' is mounted in the second bleb 170 of FIG. 8.
FIG. 10 illustrates that an additional shunt 200" is mounted in the first bleb 140 of FIG. 8.

### [Mode for Invention]

Reference will now be made in detail to embodiments of the disclosure, examples of which are illustrated in the accompanying drawings. Wherever possible, the same reference numbers will be used throughout the drawings to refer to the same or like parts. In general, a suffix such as "module" and "unit" may be used to refer to elements or components. Use of such a suffix herein is merely intended to facilitate description of the present disclosure, and the suffix itself is not intended to give any special meaning or function. The accompanying drawings are used to help easily understand various technical features and it should be understood that embodiments presented herein are not limited by the accompanying drawings. As such, the present disclosure should be construed to extend to any alterations, equivalents and substitutes in addition to those which are particularly set out in the accompanying drawings.

The terms including an ordinal number such as first, second, or the like may be used to describe various components, but the components are not limited by such terms. The terms are used only for the purpose of distinguishing one component from other components.

When any component is described as "being connected" or "being coupled" to other component, this should be understood to mean that another component may exist between them, although any component may be directly connected or coupled to the other component. In contrast, when any component is described as "being directly connected" or "being directly coupled" to other component, this should be understood to mean that no component exists between them.

A singular expression can include a plural expression as long as it does not have an apparently different meaning in context.

In the present disclosure, terms "include" and "have" should be understood to be intended to designate that illustrated features, numbers, steps, operations, components, parts or combinations thereof are present and not to preclude the existence of one or more different features, numbers, steps, operations, components, parts or combinations thereof, or the possibility of the addition thereof.

In the drawings, sizes of the components may be exaggerated or reduced for convenience of explanation. For example, the size and the thickness of each component illustrated in the drawings are arbitrarily illustrated for convenience of explanation, and thus the present disclosure is not limited thereto unless specified as such.

If any embodiment is implementable differently, a specific order of processes may be performed differently from the order described. For example, two consecutively described processes may be performed substantially at the same time, or performed in the order opposite to the described order.

In the following embodiments, when layers, areas, components, or the like are connected, the following embodiments include both the case where layers, areas, and components are directly connected, and the case where layers, areas, and components are indirectly connected to other layers, areas, and components intervening between them.

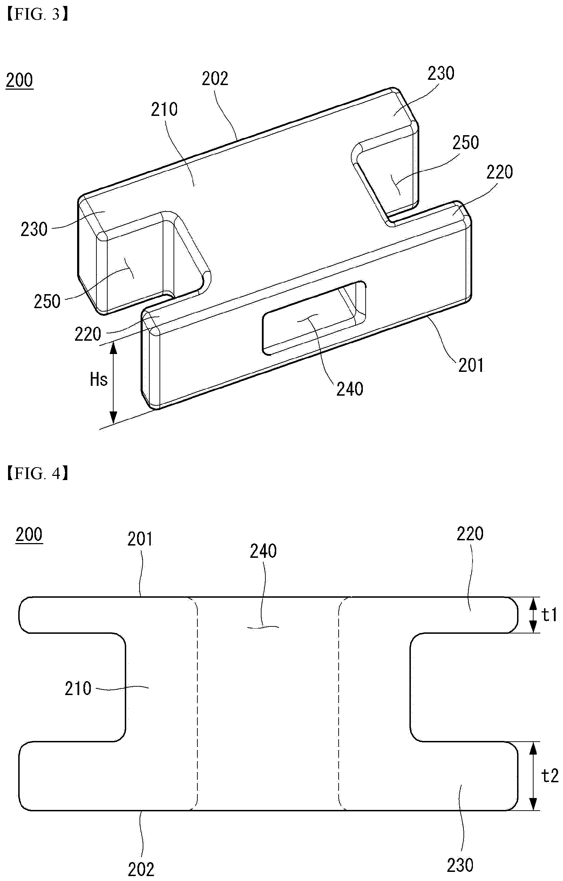

FIG. 2 illustrates that a shunt 200 according to an embodiment of the present disclosure is mounted in the first bleb 140 of FIG. 1. FIG. 3 is a perspective view illustrating the shunt 200 of FIG. 2.

The first bleb 140 may be formed around a glaucoma drainage device 150. The first bleb 140 may include aqueous humor drained from an anterior chamber 110 or the glaucoma drainage device 150. The first bleb 140 may be formed in a convex shape protruding from a sclera 120.

The first bleb 140 may include a first fibrous encapsulation 141 surrounding the first bleb 140. The first fibrous encapsulation 141 may be formed as an encapsulation of the first bleb 140 is fibrosed.

The first bleb 140 may include a first internal space 142 formed between the first fibrous encapsulation 141 and the sclera 120. The aqueous humor may be filled in the first internal space 142.

Depending on a location of the glaucoma drainage device 150 or conditions of a patient, the first bleb 140 may convexly protrude on the sclera 120. The first internal space 142 may be formed between the sclera 120 and the first fibrous encapsulation 141.

The shunt 200 may extend from an end 201 to another end 202. For example, the end 201 of the shunt 200 may be a front end of the shunt 200. For example, the other end 202 of the shunt 200 may be a rear end of the shunt 200. A longitudinal direction of the shunt 200 may be parallel to a direction from the end 201 to the other end 202. For example, the longitudinal direction of the shunt 200 may be a front-rear direction.

The end 201 of the shunt 200 may be directed toward the outside of the first bleb 140. The end 201 of the shunt 200 may be positioned outside the first fibrous encapsulation 141. The other end 202 of the shunt 200 may be directed toward the inside of the first bleb 140. The other end 202 of the shunt 200 may be positioned inside the first fibrous encapsulation 141. The other end of the shunt 200 may be positioned in the first internal space 142.

The shunt 200 may include a main body 210. The main body 210 may extend in the longitudinal direction of the shunt 200. The main body 210 may be formed in a rectangular parallelepiped shape. The main body 210 may be arranged to penetrate the first fibrous encapsulation 141. A lower surface (lower side in FIG. 3) of the main body 210 may be arranged to face the sclera 120.

The main body 210 may be formed longer than a thickness of the first fibrous encapsulation 141. Hence, a side of the main body 210 may be located outside the first fibrous encapsulation 141, and another side of the main body 210 may be located inside the first fibrous encapsulation 141. A width of the main body 210 may be greater than a height of the main body 210.

The shunt 200 may include a first support member 220. The first support member 220 may be connected to the side of the main body 210. The first support member 220 may be located outside the first bleb 140. The first support member 220 may be located outside the first fibrous encapsulation 141.

The first support members 220 may be respectively formed on opposite sides of the main body 210 facing each other in a width direction of the main body 210. The first support members 220 may extend in the width direction of the main body 210 on the opposite sides of the main body 210. The first support members 220 may extend in a width direction of the first bleb 140 on the opposite sides of the main body 210. The first support members 220 may be caught on an outer surface of the first fibrous encapsulation 141 to prevent the shunt 200 from falling into the inside of the first fibrous encapsulation 141. The front of the main body 210 may be supported on the outer surface of the first fibrous encapsulation 141 by the first support members 220.

The first support member 220 may be located between the first fibrous encapsulation 141 and the conjunctiva 130. The first support member 220 may be in contact with the outer surface of the first fibrous encapsulation 141. The first support member 220 may be in contact with the conjunctiva 130.

The shunt 200 may include a second support member 230. The second support member 230 may be connected to the other side of the main body 210. The second support member 230 may be located inside the first bleb 140. The second support member 230 may be located inside the first fibrous encapsulation 141.

The second support members 230 may be respectively formed on opposite sides of the main body 210 facing each other in the width direction of the main body 210. The second support members 230 may extend on the opposite sides of the main body 210 in the width direction of the main body 210. The second support members 230 may extend on the opposite sides of the main body 210 in the width direction of the first bleb 140. The second support members 230 may be caught on an inner surface of the first fibrous encapsulation 141 to prevent the shunt 200 from falling into the outside of the first fibrous encapsulation 141. The rear of the main body 210 may be supported on the inner surface of the first fibrous encapsulation 141 by the second support members 230.

The second support member 230 may be in contact with the inner surface of the first fibrous encapsulation 141. The second support member 230 may extend in a direction parallel to the direction in which the first support member 220 extends.

A height HS of the shunt 200 may be less than a height of the glaucoma drainage device 150. In case that the top of the shunt 200 is formed flat, the height of the main body 210, a height of the first support member 220, and a height of the second support member 230 may be the same as the height HS of the shunt 200.

The shunt 200 may include a flow path 240 formed in the main body 210. The flow path 240 may be formed by penetrating the main body 210 in the longitudinal direction. For example, the flow path 240 may be formed by penetrating the main body 210 in the front-rear direction. For example, the flow path 240 may be formed as a rectangular hole. For example, the flow path 240 may be formed as an oval hole. For example, the flow path 240 may be formed as a semi-oval hole. The flow path 240 may be formed sufficiently wide so that the aqueous humor within the first bleb 140 can be drained to the outside of the first bleb 140 through the flow path 240.

A width of the flow path 240 may greater than a height of the flow path 240. The width of the flow path 240 may be formed sufficiently long within a range where the center of the flow path 240 is not pressed by an external force acting on the shunt 200. The external force acting on the shunt 200 may include a force of surrounding tissue of the shunt 200, a force blinking the eyeball 100, or the like. Through the shape of the flow path 240, the height of the flow path 240 can be minimized and a cross-sectional area of the flow path 240 can increase. As a result, the height of the main body 210 in which the flow path 240 is formed can also be minimized, and the aqueous humor in the first bleb 140 can be smoothly drained through the flow path 240.

The inside of the first bleb 140 and the outside of the first bleb 140 may be connected by the flow path 240. Hence, the aqueous humor of the first internal space 142 can be drained to the outside of the first bleb 140. For example, the first internal space 142 may be connected to a space formed between the first bleb 140 and the conjunctiva 130 by the flow path 240. Because the aqueous humor of the first internal space 142 is in a high pressure state, the aqueous humor may be drained to a low pressure side through the flow path 240. For example, the aqueous humor of the first internal space 142 may be drained to the space formed between the first bleb 140 and the conjunctiva 130.

The shunt 200 may include an installation groove 250 formed by being surrounded by the main body 210, the first support member 220, and the second support member 230. Both sides of the installation groove 250 may be open in a height direction (up-down direction) of the shunt 200. The installation groove 250 may be formed so that the opposite side of the flow path 240 is open in a width direction of the shunt 200. The first fibrous encapsulation 141 may be accommodated in the installation groove 250. Hence, the first support member 220 located outside the first fibrous encapsulation 141 may not enter the first internal space 142. As a result, the second support member 230 located inside the first fibrous encapsulation 141 may not fall out of the first bleb 140.

Edges of the shunt 200 may be rounded. The rounding can minimize damage to the conjunctiva 130 caused by the edges of the shunt 200. If the conjunctiva 130 is damaged, fine tissues within the conjunctiva 130 may be stimulated, thereby promoting ulceration or fibrosis.

Edges of the flow path 240 may be rounded. The rounding can improve an impact strength or a fracture resistance around the flow path 240.

The shunt 200 may be formed by including a silicone material. The main body 210, the first support member 220, and the second support member 230 may be formed by including a silicone material. Hence, the flexibility of the shunt 200 can be improved, and a foreign body sensation caused by the shunt 200 when the eyeball 100 or the eyelid moves can be reduced. In addition, when an impact is applied to the eyeball 100 or the area around the eyeball 100, the risk of the eyeball 100 being damaged by the shunt 200 can be reduced. In addition, because the shunt 200 is easy to be installed and removed, the shunt 200 can be installed and removed with minimum procedures in a short period of time.

The shunt 200 may be formed by including a sterilized silicone material. For example, the shunt 200 may be formed by including a medical grade implantable silicone material that can be implanted into the human body. For example, the shunt 200 may be formed by including MED-4830 manufactured by Nusil Technology LLC. As a result, the stability of the shunt 200 in the body can be improved, and a rejection reaction to the shunt 200 can be reduced.

FIG. 4 is a plan view of the shunt 200 of FIG. 3. A thickness of the first support member 220 is a length of the first support member 220 and may be referred to as a first thickness t1. A thickness of the second support member 230 is a length of the second support member 230 and may be referred to as a second thickness t2.

The first thickness t1 may be less than the second thickness t2. Hene, the foreign body sensation caused by the first support member 220 located outside the first bleb 140 can be reduced.

The second thickness t2 may be thicker than the first thickness t1. Hence, the second support member 230 located inside the first bleb 140 may have a difficulty falling out of the first fibrous encapsulation 141. If the second thickness t2 is too thin, the second support member 230 may easily fold toward the other end 202 of the main body 210. Therefore, the second support member 230 may fold and fall out of the first bleb 140 due to a pressure inside the first bleb 140 or the external force applied to the shunt 200.

To make it difficult for the second support member 230 to fall out of the first fibrous encapsulation 141, the second support member 230 may sufficiently extend from the main body 210.

In other embodiments, the first thickness t1 may be similar to the second thickness t2. Hene, it can be difficult for the first support member 220 to enter the inside of the first fibrous encapsulation 141. In addition, the shape of the first support member 220 and the shape of the second support member 230 are similar, and thus the inside and the outside of the shunt 200 may be changed and used.

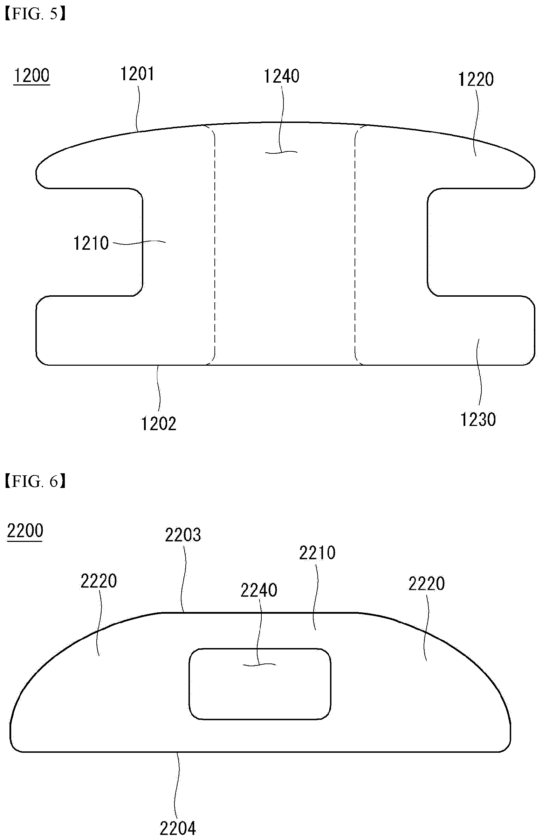

FIG. 5 is a plan view illustrating a shunt 1200 according to another embodiment of the present disclosure.

A front surface of a main body 1210 may get closer to a rear surface of the main body 1210 as it goes away from the center of the main body 1210. For example, the front surface of the main body 1210 may be formed in a curved shape.

In addition, a front surface of the first support member 1220 may get closer to a rear surface of the first support member 1220 as it goes away from the main body 1210. For example, the front surface of the first support member 1220 may be formed in a curved shape and may be continuously formed by being connected to the front surface of the main body 1210.

The front surface of the main body 1210 and the front surface of the first support member 1220 may be formed in a curved shape which convexly protrudes as they go to the center of the shunt 1200. A front surface of the shunt 1200 may be formed in a convex shape.

As a result, a foreign body sensation caused by the front surface of the shunt 1200 can be reduced. In addition, damage to the conjunctiva 130 (see FIG. 2) caused by a front side of the main body 1210 and the first support member 1220 can be minimized.

FIG. 6 is a front view illustrating a shunt 2200 according to another embodiment of the present disclosure.

An upper surface of a first support member 2220 may get closer to the sclera 120 (see FIG. 2) as it goes away from a main body 2210. For example, the upper surface of the first support member 2220 may be formed in a curved surface and connected to an upper surface of the main body 2210. A connection part between the upper surface of the first support member 2220 and the upper surface of the main body 2210 may be rounded.

An upper surface of a front of the main body 2210 may get closer to the sclera 120 as it goes away from the center of the main body 2210 in a width direction. For example, the upper surface of the main body 2210 may be formed in a curved surface and may be continuously formed by being connected to the upper surface of the first support member 2220.

Because the upper surface of the front of the main body 2210 and the upper surface of the first support member 2220 are positioned close to the conjunctiva 130 (see FIG. 2), damage to the conjunctiva 130 (see FIG. 2) can be minimized by the shapes of the main body 2210 and the first support member 2220. In addition, a foreign body sensation caused by the main body 2210 and the first support member 2220 can be reduced.

In addition, a second support member (not shown, see '230' of FIG. 3) may be formed in the same or similar manner as the first support member 2220. The upper surface of the main body 2210 may be entirely formed in the same or similar manner as the upper surface of the front of the main body 2210. An upper surface of the second support member may be continuously formed by being connected to an upper surface of a rear of the main body 2210.

The second support member may be positioned close to an inner surface of the first bleb 140 (see FIG. 2), and the main body 2210 may penetrate the first bleb 140. Therefore, damage to the first bleb 140 can be minimized by the shape of the second support member and the main body 2210.

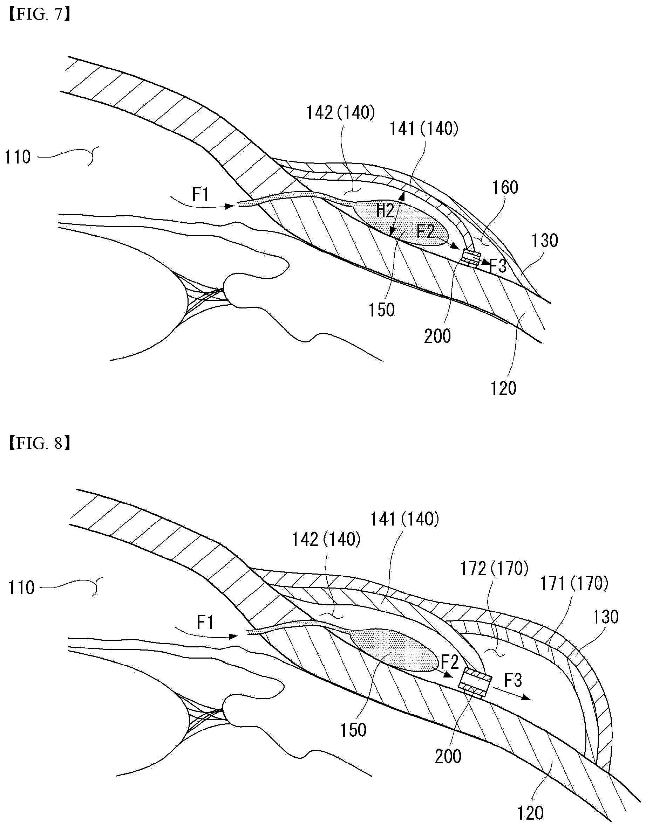

When the shunt 200 is mounted in the first bleb 140 as illustrated in FIG. 2, the aqueous humor of the first internal space 142 may be drained to the outside of the first bleb 140 as illustrated in FIG. 7. For example, the aqueous humor of the first internal space 142 may be drained to a subconjunctival space 160 through the shunt 200. A direction in which the aqueous humor of the first internal space 142 is drained to the outside of the first bleb 140 (e.g., the subconjunctival space 160) through the shunt 200 may be referred to as a third direction F3. The aqueous humor of the anterior chamber 110 may move through the first direction F1, the second direction F2, and the third direction F3. When the aqueous humor within the first bleb 140 is drained, a pressure increase caused by the aqueous humor can be alleviated, and thus a pressure of the first bleb 140 and a pressure (intraocular pressure) of the eyeball 100 (see FIG. 1) can be reduced.

When the shunt 200 is mounted in the first bleb 140, the shunt 200 may be mounted as close as possible to a connection part between the first bleb 140 and the sclera 120. Hence, the foreign body sensation and damage to the conjunctiva 130 caused by the shunt 200 can be minimized. In addition, when the shunt 200 is mounted in the first bleb 140, the upper surface of the shunt 200 may be mounted lower than an upper surface of the glaucoma drainage device 150. Hence, the foreign body sensation and damage to the conjunctiva 130 caused by the shunt 200 can be minimized.

A height of the first internal space 142 in FIG. 2 may be referred to as a first height H1, and a height of the first internal space 142 in FIG. 7 may be referred to as a second height H2. The second height H2 may be lower than the first height H1. When the aqueous humor in the first bleb 140 is drained to the outside of the first bleb 140 by the shunt 200, the first height H1 may be lowered to become the second height H2. As a result, the pressure in the first bleb 140 can be reduced.

If the aqueous humor in the first bleb 140 continues to flow in the third direction F3, a new bleb, which is a second bleb 170, may be formed outside the shunt 200, as illustrated in FIG. 8. The aqueous humor drained to the outside of the first bleb 140 may be widely spread by the second bleb 170 and absorbed into the blood flow.

As time passes, the second bleb 170 may be fibrosed and may not smoothly absorb the aqueous humor. The shunt 200 may be additionally mounted in the first bleb 140 or the second bleb 170 to drain the aqueous humor within the first bleb 140 or the second bleb 170. The additionally mounted shunt 200 may be mounted at a different location from the previously mounted shunt 200.

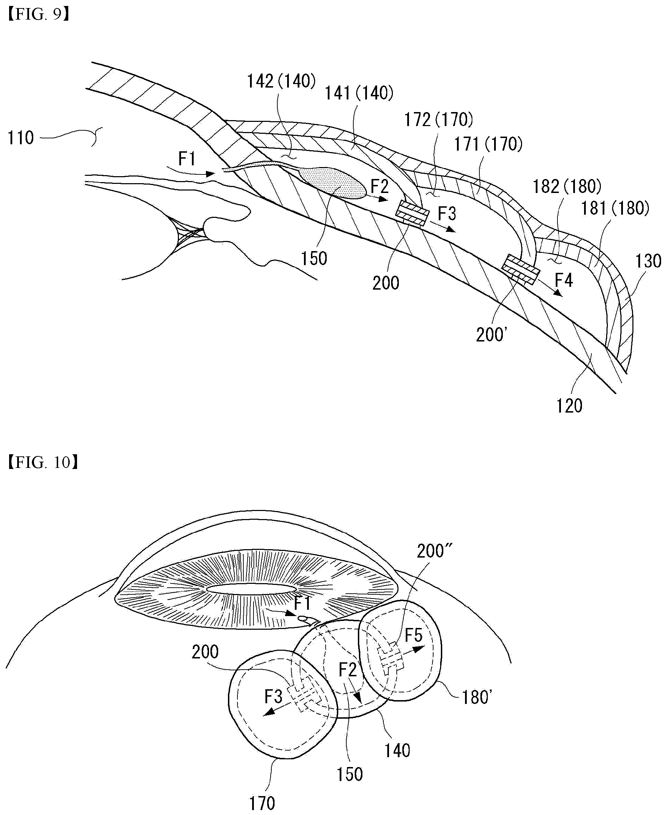

FIG. 9 illustrates that an additional shunt 200' is mounted in the second bleb 170 of FIG. 8. The additional shunt 200' may be mounted in the second bleb 170. For example, the additional shunt 200' may be mounted in a second fibrous encapsulation 171 of the second bleb 170.

The aqueous humor in the second bleb 170 may be drained to the outside of the second bleb 170 by the additional shunt 200'. For example, the aqueous humor in a second internal space 172 may be drained to the space between the conjunctiva 130 and the sclera 120 by the additional shunt 200'. A direction in which the aqueous humor in the second bleb 170 is drained to the outside of the second bleb 170 by the additional shunt 200' may be referred to as a fourth direction F4. The fourth direction F4 may be a direction parallel to the third direction F3. The aqueous humor in the anterior chamber 110 may move through the first direction F1, the second direction F2, the third direction F3, and the fourth direction F4.

If the aqueous humor in the second bleb 170 continues to flow in the fourth direction F4, a new bleb, which is a third bleb 180, may be formed outside the additional shunt 200'. The aqueous humor drained from the second bleb 170 to the third bleb 180 may be widely spread by the third bleb 180 and absorbed into the blood flow. As time passes, the third bleb 180 may be fibrosed. Further, a third fibrous encapsulation 181 may be formed, and the aqueous humor may accumulate in a third internal space 182.

FIG. 10 illustrates that an additional shunt 200" is mounted in the first bleb 140 of FIG. 8. The additional shunt 200" may be mounted in the first bleb 140. For example, the shunt 200 and the additional shunt 200" may be arranged such that the glaucoma drainage device 150 are located between the shunt 200 and the additional shunt 200". For example, the shunt 200 and the additional shunt 200" may be positioned along the perimeter of the cornea. For example, if the shunt 200 is mounted at the 9 o'clock direction of the first bleb 140 (left side in FIG. 10), the additional shunt 200" may be mounted at the 3 o'clock direction of the first bleb 140 (right side in FIG. 10). For example, if the shunt 200 is mounted at the 9 o'clock direction of the first bleb 140 (left side in FIG. 10), the additional shunt 200" may be mounted at the 6 o'clock direction of the first bleb 140 (lower side in FIG. 10).

The aqueous humor in the first bleb 140 may be drained to the outside of the first bleb 140 by the additional shunt 200". For example, the aqueous humor in the first internal space 142 may be drained to the space between the conjunctiva 130 and the sclera 120 by the additional shunt 200".

A direction in which the aqueous humor in the first bleb 140 is drained to the outside of the first bleb 140 by the additional shunt 200" may be referred to as a fifth direction F5. The fifth direction F5 may form an angle with respect to the third direction F3. For example, the fifth direction F5 may be the opposite direction to the third direction F3. The aqueous humor in the anterior chamber 110 may move through the first direction F1, the second direction F2, and the third direction F3 or may move through the first direction F1, the second direction F2, and the fifth direction F5.

If the aqueous humor in the first bleb 140 continues to flow in the fifth direction F5, a new bleb, which is a third bleb 180', may be formed outside the additional shunt 200". The aqueous humor drained from the first bleb 140 to the third bleb 180' may be widely spread by the third bleb 180' and absorbed into the blood flow.

The blebs 140, 170 and 180 may indicate at least one of the first bleb 140, the second bleb 170, and the third bleb 180. The plurality of shunts 200 may be mounted in the blebs 140, 170 and 180 which are fibrosed and have a high internal pressure.

A surgical method of installing the shunt 200 according to an embodiment of the present disclosure to the first bleb 140 formed around the glaucoma drainage device 150 is described below with reference to FIG. 2. The shunt 200 can also be applied to a bleb formed by trabeculectomy, in addition to the blebs 140, 170 and 180 formed by the glaucoma drainage device 150.

A surgical site may be prepared through a general anesthesia process and sterilization process. The surgical site may be exposed using drugs or stay sutures to secure a surgical view.

After checking the extent of the fibrosed first bleb 140, a site where the shunt 200 is to be installed may be determined depending on a location of the glaucoma drainage device 150 of a patient. If the glaucoma drainage device 150 is installed on a lateral side of the eyeball 100, an upward site or a downward site of the first bleb 140 may be determined as the site where the shunt 200 is to be installed. The site where the shunt 200 is to be installed may be determined as a site as close to the sclera 120 as possible.

The conjunctiva 130 may be incised to expose the first bleb 140. For example, the conjunctiva 130 at the site where the shunt 200 is installed may be held with conjunctival forceps and may be bluntly dissected with Stevens or Westcott tenotomy scissors to expose a part of the first bleb 140. The conjunctiva 130 may be incised while avoiding blood vessels of the conjunctiva 130. However, if it is difficult to avoid the blood vessels of the conjunctiva 130, the conjunctiva 130 may be incised after a hemostatic treatment, such as cauterization, and hemostatic treatment can be applied to any bleeding that occurs during the incision.

A part of the first fibrous encapsulation 141 of the exposed first bleb 140 may be incised or excised to provide a space for installing the shunt 200. For example, a part of the exposed first fibrous encapsulation 141 may be incised or excised using a blade or a special punch to provide a space for installing the shunt 200. In this instance, the first fibrous encapsulation 141 may be incised longer than a widthwise length of the shunt 200.

The shunt 200 may be mounted in an incision site of the first fibrous encapsulation 141. For example, the shunt 200 may be implanted into the incision site of the first fibrous encapsulation 141 using forceps and mounted in the first fibrous encapsulation 141. In this instance, the second support member 230 may be made to enter the first internal space 142 inside the first bleb 140.

If a length of the incision site of the first fibrous encapsulation 141 is excessively longer than a width of the shunt 200, the incision site of the first fibrous encapsulation 141 may be sutured using a suture after the shunt 200 is mounted.

After the shunt 200 is installed, it may be confirmed that the aqueous humor in the first bleb 140 is drained to the outside of the first bleb 140 through the shunt 200, and the conjunctiva 130 may be sutured using the suture. As described above, because the surgery to install the shunt 200 in the first fibrous encapsulation 141 is performed minimally invasively, the physical and financial burden on the patient for the surgery can be reduced, and the patient's recovery speed can be accelerated. In addition, because the damage to the first bleb 140 is minimized, the possibility of conjunctival ulceration can be reduced.

Some embodiments or other embodiments of the present disclosure described above are not mutually exclusive or distinct from each other. Configurations or functions of some embodiments or other embodiments of the present disclosure described above can be used together or combined with each other.

It is apparent to those skilled in the art that the present disclosure can be embodied in other specific forms without departing from the spirit and essential features of the present disclosure. Accordingly, the above detailed description should not be construed as limiting in all aspects and should be considered as illustrative. The scope of the present disclosure should be determined by rational interpretation of the appended claims, and all modifications within an equivalent scope of the present disclosure are included in the scope of the present disclosure.

## Claims

1. A shunt comprising:
a main body penetrating a fibrous encapsulation of a bleb formed between a sclera and a conjunctiva; and
a flow path penetrating the main body, the flow path draining an aqueous humor within the bleb to an outside of the bleb.

2. The shunt of claim 1, further comprising:
a first support member connected to a front of the main body and positioned outside the fibrous encapsulation; and
a second support member connected to a rear of the main body and positioned inside the fibrous encapsulation,
wherein the main body includes a lower surface facing the sclera, and
wherein the flow path penetrates the main body from the front to the rear.

3. The shunt of claim 1, wherein a width of the main body is greater than a height of the main body.

4. The shunt of claim 1, wherein a height of the main body is less than a height of a glaucoma drainage device located inside the bleb.

5. The shunt of claim 1, wherein a width of the flow path is greater than a height of the flow path.

6. The shunt of claim 2, wherein the front of the main body is supported on an outer surface of the fibrous encapsulation by the first support member, and
wherein the rear of the main body is supported on an inner surface of the fibrous encapsulation by the second support member.

7. The shunt of claim 2, wherein the first support member extends in a width direction of the main body, and
wherein the second support member extends in the width direction of the main body.

8. The shunt of claim 7, wherein the fibrous encapsulation is implanted between the first support member and the second support member.

9. The shunt of claim 1, wherein the main body is located adjacent to a connection part between the bleb and the sclera.

10. The shunt of claim 2, wherein, in a longitudinal direction of the main body, the first support member is thinner than the second support member.

11. The shunt of claim 2, wherein, in a longitudinal direction of the main body, a thickness of the first support member is similar to a thickness of the second support member.

12. The shunt of claim 2, wherein a front surface of the first support member includes a curved surface which gets closer to a rear surface of the first support member as it goes away from the main body.

13. The shunt of claim 2, wherein an upper surface of the first support member and an upper surface of the second support member each include a curved surface which gets closer to the sclera as it goes away from the main body.

14. The shunt of claim 13, wherein an upper surface of the main body includes a curved surface which gets closer to the sclera as it goes away from a center of the main body in a width direction, and
wherein the curved surface of the main body is formed continuously with the curved surface of the first support member and the curved surface of the second support member.

15. The shunt of claim 2, wherein the main body, the first support member, and the second support member each include a rounded edge.

16. The shunt of claim 1, wherein the flow path includes a rounded edge.

17. The shunt of claim 1, further comprising a first shunt and a second shunt respectively mounted at different locations of the fibrous encapsulation.

18. The shunt of claim 17, wherein the first shunt and the second shunt are arranged such that a glaucoma drainage device inside the bleb is located between the first shunt and the second shunt.

19. The shunt of claim 18, wherein the first shunt and the second shunt are arranged along a perimeter of a cornea.

20. The shunt of claim 1, further comprising:
a first shunt mounted in the fibrous encapsulation of the bleb; and
a second shunt formed by the aqueous humor drained to the outside of the bleb, the second shunt being mounted in a fibrous encapsulation of a second bleb connected to the bleb.
